# EUROPEAN PATENT APPLICATION

(11) **EP 3 987 942 A1**
(43) Date of publication of application: **27.04.2022**
(21) Application number: 20203915.2
(22) Date of filing: 26.10.2020
(51) Int. Cl.: A23L 19/00, A23L 33/105, C11B 1/06

(54) **OLIVE-DERIVED COMPOSITIONS**

(71) Applicant: Mediakos GmbH, 10115 Berlin (DE)
(72) Inventor: The designation of the inventor has not yet been filed
(74) Representative: Dentons Patent Solutions Rechtsanwaltsgesellschaft mbH

(57) **Abstract**

The present invention relates to a method of producing at least one composition, said method comprising the following steps: (a) preparing a paste from flesh of fermented Kalamon olives; (b) subjecting said paste to a separation process yielding oil, a semisolid fraction, and an aqueous phase, said separation process preferably being centrifugation; and (c) performing one, two or three of the following: (i) harvesting said oil, thereby obtaining a first composition; (ii) drying said semisolid fraction, thereby obtaining a second composition; and (iii) harvesting said aqueous phase, thereby obtaining a third composition.

## Description

The present invention relates to a method of producing at least one composition, said method comprising the following steps: (a) preparing a paste from flesh of fermented Kalamon olives; (b) subjecting said paste to a separation process yielding oil, a semisolid fraction, and an aqueous phase, said separation process preferably being centrifugation; and (c) performing one, two or three of the following: (i) harvesting said oil, thereby obtaining a first composition; (ii) drying said semisolid fraction, thereby obtaining a second composition; and (iii) harvesting said aqueous phase, thereby obtaining a third composition.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Olives and olive oil have been described to modulate lipid metabolism. The influence of olive species, the geographic region of origin and treatment of olives have not yet received sufficient attention when it comes to optimizing the beneficial effects of olive-derived products. Furthermore, the prior art fails to teach which specific fractions of blood cholesterol may be beneficially modulated. In addition, the prior art fails to consider pre-treatment of olives prior to obtaining any products from olives.

While certain individuals may appreciate the taste of olives, this does not apply to all individuals who might benefit from the advantageous properties of a frequent consumption of olives.

The above described state of the art as well as deficiencies thereof have been recognized by the present inventors.

The technical problem underlying the present invention can be seen in the provision of improved means and methods of modulating lipid metabolism.

This technical problem has been solved by the enclosed claims.

Accordingly, in the first aspect, the present invention relates to a method of producing at least one composition, said method comprising the following steps: (a) preparing a paste from flesh of fermented Kalamon olives; (b) subjecting said paste to a separation process yielding oil, a semisolid fraction, and an aqueous phase, said separation process preferably being centrifugation; and (c) performing one, two or three of the following: (i) harvesting said oil, thereby obtaining a first composition; (ii) drying said semisolid fraction, thereby obtaining a second composition; and (iii) harvesting said aqueous phase, thereby obtaining a third composition.

Kalamon olives are a variety of olives. They are also referred to as Kalamata olives. The systematic name is *Olea europaea L., cv. Kalamata.*

Olive flesh of fermented Kalamon olives is transformed into a paste in accordance with step (a) of the method of the first aspect. For this purpose, preferably a hammer mill is used.

The separation process in accordance with step (b) is preferably implemented by a centrifugation procedure on a three-phase decanter. The paste yields oil, a semisolid fraction, and an aqueous phase which may also be referred to as first, second and third fraction, respectively.

In accordance with step (c), said semisolid fraction may be subjected to drying. Preferably, drying is done by a two-step dehydration procedure comprising the first step of air drying and a second step of machine-driven drying such as flow bed drying or lyophylisation.

Harvesting of oil and/or aqueous phase is straightforward and can be done, e.g. by decanting and/or pumping.

A commonly known product obtained from olives is olive oil. Of note, the requirement in accordance with the invention of the olives being fermented is not routinely met in the preparation of olive oil. This distinction renders the fractions or compositions obtained from fermented Kalamon olives different from common olive products. Moreover, fermentation not only renders the fractions, compositions and capsules of the invention distinct, but it also enhances their beneficial properties which are disclosed in more detail further below.

In particular, and having regard to said first composition or said oil in accordance with the invention, using fermented olives as starting material leads to an increase in contents of hydroxytyrosol and tyrosol. Common olive oil contains esters of these compounds such as oleacein and oleocanthal. Fermentation of the olives prior to processing in accordance with the invention entails hydrolysis of these compounds and sets free hydroxytyrosol and tyrosol. This is one of the preferred features which distinguishes the oil and the first composition obtained by the method of the invention from common olive oil.

Having regard to the aqueous phase and the third composition of the invention, it is of note that fermented olives have an elevated content of lactic acid. This renders the compositions of the invention, especially the third composition, distinct from aqueous extracts of non-fermented olives.

In a preferred embodiment of the method of the first aspect, said method further comprises (ba) washing, preferably a plurality of times such as three times, said semisolid fraction obtained in (b) with water, thereafter combining said water with said aqueous phase obtained in (b), thereby obtaining a washed semisolid fraction and a combined aqueous phase; and/or (ca) filtrating said oil obtained in (c)(i), thereby obtaining a filtrated first composition.

It is understood that washing said semisolid fraction entails the need for separating the water used for washing from said semisolid fraction which has undergone washing. This separating is preferably accomplished by filtrating.

Preferably, said filtrating, be it of the oil of or the washed semisolid fraction, is implemented by means of a filter press. This removes any insoluble material. In case of filtrating said oil, the residue on the filter is preferably discarded. In case of washing said semisolid fraction, any residue on the filter is preferably retained and combined to give rise to the washed semisolid fraction.

Repeated washing of the semisolid fraction as embraced by (ba) is exemplified in the Example.

In a further preferred embodiment, the method of the first aspect further comprises (d) encapsulating said first composition into a first capsule, wherein preferably (i) said first capsule is a soft capsule; and/or (ii) the amount of said first composition which is encapsulated corresponds to two to ten, preferably five of said olives.

In a further preferred embodiment, the method of the first aspect further comprises (e) encapsulating said second composition into a second capsule, wherein preferably (i) said second capsule is a hard capsule and/or a capsule with an enteric coating; and/or (ii) the amount of said second composition which is encapsulated corresponds to two to ten, preferably five of said olives.

In a further preferred embodiment, the method of the first aspect further comprises (f) encapsulating said third composition into a third capsule, wherein preferably (i) said third capsule is a hard capsule; and/or (ii) the amount of said third composition which is encapsulated corresponds to two to ten, preferably five of said olives.

Preferably, said third composition, prior to encapsulating it, is absorbed on microcrystalline cellulose.

In a particularly preferred embodiment, said method comprises (g) encapsulating said first capsule and said third capsule into a fourth capsule, preferably a hard capsule and/or a capsule with an enteric coating. Such fourth capsule combines beneficial agents comprised in the oil obtained in step (c)(i) with the beneficial agents comprised in the aqueous phase obtained in step (c)(iii). The same applies to any combination of first and third compositions.

In terms of beneficial effects (discussed in more detail below), second composition and capsule, respectively, are the least preferred.

The term "enteric coating" has its art established meaning. Accordingly, such coating provides for said capsule to pass the stomach and not be digested prior to reaching the small intestine.

Hard and soft capsules are known in the art. Preferred materials for hard capsules are gelatin and hydroxymethylpropyl cellulose (HMPC).

The present inventors surprisingly discovered that compositions obtained in accordance with the present invention, obtained by the methods of the invention, and being obtained from only about five olives are capable of providing the beneficial effects disclosed further below.

Also, the composition(s) obtained from three, four, six, seven, eight, or nine olives may be used and/or encapsulated as defined above.

It is apparent from the above, that it is particularly preferred to obtain compositions (or capsules) or combinations of compositions (or of capsules) which on the one hand comprise oil as defined herein above and on the other hand those constituents which are comprised in the aequous phase obtained during olive processing in accordance with the methods of the invention.

Not only is surprising that rather small amounts of olives (as stated above, 2 to 10, preferably 5 olives) provide for beneficial effects, but furthermore compliance is improved by administration of capsules to those individuals which reject olives or olive oil for reasons such as the taste thereof not being agreeable. As such, the invention provides for easier and more practical intake of the beneficial compounds comprised in fermented Kalamon olives and the fractions thereof in accordance with the invention.

In a further preferred embodiment, said method comprises prior to step (a), allowing fermentation of said olives to occur, wherein said fermentation occurs (i) for a time span of about 3 to about 12 months, preferably about 4 to about 6 months; and/or (ii) in the presence of autochthonous yeasts, e.g. as they occur on the surface of said olives, wherein preferably no further agent triggering fermentation is added.

Fermentation may also take about 1, about 2, about 4, about 5, about 7, about 8, about 9, about 10 or about 11 months.

In a further preferred embodiment, said Kalamon olives are grown in Peloponnese, preferably in Lakonia or Messinia. Surprisingly, it turns out that when olives from these regions are used, the beneficial effects of the fractions, compositions and capsules of the invention are particularly pronounced.

Preferably, said olives are harvested by hand picking. For the purpose of fermentation, they are preferably placed in brine. Preferred microorganisms catalyzing the fermentation are autochthonous yeasts from the respective region of origin of the olives. Preferred regions of origin are detailed above.

As known in the art, after fermentation, the olives are separated from the brine. They are washed and gently dried. Thereafter the stone is removed.

A preferred preparation procedure consists of the following steps; for further details see the Example.
(1) Olives, processed and debittered using the Kalamata style (see below) are obtained from the fermentation tank, washed and gently dried.
(2) The stone is removed and the flesh is blended/mashed mechanically leading to an olive paste.
(3) The olive paste is centrifuged leading to three distinct phases: Oil (A), semisolid paste (B1) and aqueous phase (C1).
(4) The semisolid paste (B1) is mixed with water and vortexed until homogenization and then filtered. This yields a filtrate (C2) and a semisolid paste (B2). Step (4) may be repeated thereby yielding further filtrates (C3, C4,...).
(5) The aqueous phase obtained after filtration (C2 and, where applicable C3, C4,...) is combined with C1 to yield the final product C.
(6) The semisolid phase B2 obtained after filtration is oven dried yielding the final product B.

The Kalamata style of processing uses black olive fruits of the olive cultivar Kalamon, which are fermented in brine (3-9% salt) for 3-6 months with the autochthonous yeasts found on the surface of the fruit and maintaining a pH value that guarantees lactic fermentation.

In a second aspect, the present invention provides one or more compositions or capsules selected from: (i) a combination of said first and said third composition, both obtained by the method of any one of the preceding claims or the fourth capsule obtained by the method of claim 6; (ii) the first composition or capsule obtained by the method of any one of the preceding claims; (iii) the second composition or capsule obtained by the method of any one of the preceding claims; and (vi) the third composition or capsule obtained by the method of any one of the preceding claims.

Particularly preferred is that said third composition or capsule comprises phenolic compounds such as hydroxytyrosol and tyrosol, wherein preferably each of hydroxytyrosol and tyrosol is present in a mass ratio of 1 mg/g to 20 mg/g. This is generally inherent to the third composition as obtained by the methods of the invention.

Also particularly preferred is that said second composition or capsule comprises triterpenic compounds such as maslinic acid and oleanolic acid, wherein preferably each of maslinic acid and oleanolic acid is present in a mass ratio of 0.1 mg/g to 5 mg/g. Having said that, and as stated above, no particular preference is given to said second composition or capsule.

Beneficial effects of olive oil are known from the art, including effects on the lipid metabolism. A common denominator of the prior art is that compounds with antioxidative properties as comprised in olives, in particular phenolic compounds, are held responsible for beneficial effects on lipid metabolism.

The present inventors surprisingly discovered that this is not the case. This has been further explored by allowing phenolic compounds to oxidise and thereafter manufacture the compositions in accordance with the present invention. Also, for such compositions, the beneficial effects as disclosed further below have been confirmed.

Therefore, the present invention relates to a composition comprising all or substantially all ingredients of olives or fractions thereof, said olives being defined as herein above, and said fractions being obtained by the method of the invention as disclosed above, provided that said composition has a significantly lower concentration of phenolic compounds as compared to said olives or is essentially free of said phenolic compounds.

Preferably, said composition or capsule is a food supplement.

A food supplement has to be held distinct from a pharmaceutical composition. For the purposes of the present disclosure, a food supplement is inherently non-therapeutic in nature. Food supplements do not require a doctor's prescription. Rather, to the contrary, they can be purchased by anyone, for example in a supermarket.

The compositions of the present invention may also be used for medical purposes. As such, the present invention also relates to the use of the above disclosed fractions, compositions or capsules for use in medicine. Related thereto, provided are pharmaceutical compositions comprising or consisting of said fractions, compositions or capsules.

The fractions, compositions and capsules are particularly useful for treating dyslipidemia. Dyslipidemia is a disorder of lipoprotein metabolism, including lipoprotein overproduction or deficiency. Dyslipidemias may comprise elevation of the total cholesterol, the low-density lipoprotein (LDL) cholesterol and the triglyceride concentrations, and a decrease in the high-density lipoprotein (HDL) cholesterol concentration in the blood.

Dyslipidemia comes under consideration in many situations including diabetes, a common cause of hyperlipidemia. For adults with diabetes, it has been recommended that the levels of LDL, HDL, and total cholesterol, and triglyceride be measured every year. Optimal LDL cholesterol levels for adults with diabetes are less than 100 mg/dL (2.60 mmol/L), optimal HDL cholesterol levels are equal to or greater than 40 mg/dL (1.02 mmol/L), and desirable triglyceride levels are less than 150 mg/dL (1.7 mmol/L).

In line therewith, and in a third aspect, the invention provides such fraction, composition or capsule for use in a method of treating (a) dislipidemia; (b) disorders associated with elevated levels of LDL cholesterol; (c) disorders associated with elevated levels of total cholesterol; and/or (d) disorders associated with low levels of HDL cholesterol. Deviant levels ("elevated"; "low") are defined with respect to the desired values given above.

In a preferred embodiment, (a) said levels are concentrations in serum, blood or plasma; (b) said elevated levels of LDL cholesterol are serum concentrations, preferably between about 200 mg/dl and about 300 mg/dl; (c) said elevated levels of total cholesterol are serum concentrations above about 200 mg/dl serum, preferably between about 200 mg/dl and about 300 mg/dl serum, more preferably between about 215 mg/dl and about 275 mg/dl serum; and/or (d) the individual to be treated does not receive any further medication for the treatment of hypercholesterolemia such as statins.

The term "about" in the context of this invention refers to deviations from the recited values as they are commonly observed in the art, such as in the context of blood analysis. Exemplary deviations are +/-20%, +/-10% and +/-5%.

In a further preferred embodiment of medical uses in accordance with the present invention, said disorders are selected from cardiovascular diseases, atherosclerosis, cardiac infarct, stroke, thrombosis and embolism. Also included is low to moderate cardiovascular risk, preferably heart scores below 5%. "Score" in this context refers to Systematic COronary Risk Evaluation (SCORE): high and low cardiovascular risk charts based on gender, age, total cholesterol, systolic blood pressure and smoking status, with relative risk chart, qualifiers and instructions.

Related to the food supplement in accordance with the fourth aspect, the present invention provides, in a seventh aspect, the use of the food supplement of the invention for lowering (a) LDL cholesterol; (b) lowering total cholesterol; and/or (c) increasing HDL cholesterol in an individual.

As noted above, (a) said use is non-therapeutic; and/or (b) said individual does not suffer from and preferably is not at risk to develop any of the disorders defined herein above.

The administration of compositions of the invention does not trigger significant changes in serum glucose transaminases, urea and creatinine. Altogether, there is no indication that consumption of compositions in accordance with the present invention would have side effects.

Regardless of whether the use is therapeutic or non-therapeutic, a preferred daily dosage is one capsule. As noted above, one capsule preferably comprises material obtained from 2 to 10, preferably from 5 olives. As a consequence, and to the extent compositions in accordance with the present invention are not formulated into capsules, preferred dosages are such that daily administration of the material derived from 2 to 10, preferably 5 olives is achieved.

As regards the time period of administration, there are no particular limits. To the contrary, life long intake is possible and recommended. Beneficial effects occur after an administration for about one, two or three months.

Generally speaking, the above suggested dosages and dosage regiments will not require modification depended on age, sex, weight etc. of the individual to whom compositions are to be administered. Having said that, and to the extent such modifications are nevertheless perceived as being adequate, they can be done by the attending doctor or physician (in case of medical uses) without further ado. As noted above, an amount of compositions of the invention corresponding to about 5 olives is preferred.

As regards the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The examples illustrate the invention.

### Example 1

### Manufacture of compositions of the invention

50 olive fruits are washed and dried gently before weighing. Then, the stones are separated from the flesh and the flesh is weighed.

### Weighing results:

50 whole olive fruits: 253.5 gr (average weight of each fruit: 4.71 gr)
50 olive stones: 44.4 gr
Fruit flesh from 50 olives: 201.0 gr

After weighing, the flesh is mashed using a blender until an olive paste is obtained. The paste is then centrifuged for 10 min at 4.000 rpm. After centrifugation, the olive paste is found separated into 3 layers (phases), i.e., aqueous phase, lipid phase and a semisolid paste in the middle.

The upper layer consists of olive oil, which is analyzed using the protocol described in Karkoula et al., Journal of Agricultural and Food Chemistry, 2012, 60, 11696-11170. The total weight is 13.5 gr. Olive oil analysis shows the presence of tyrosol and hydroxytyrosol in relatively low concentrations. "Low concentrations" in this context refers to concentrations relative to those in olive flesh as well as to those in aqueous extracts according to the invention.

The olive fruit paste stays in the middle layer of the 3-layered material as obtained after the centrifugation. By gently scraping the surface, any olive oil residues that may be present on the upper side of the paste are removed. Then, the paste is stored separately from the liquid phases for further processing as described below. Olive fruit paste after the centrifugation, total weight: 151.3 gr

The lower phase of the 3-layered material consists of the aqueous phase originally comprised in the olive flesh. Total weight before drying: 22.7 gr
The olive fruit paste is divided into 50 mL falcon tubes and equal amount of distilled water to the amount of fruit paste is added in each of them. For resuspending, a heavy duty vortexer is used, for 1 min, until homogenization. The mixture is filtered using paper filter. This step is repeated with fresh water for two more times. All the water from these extractions, including the water from the step of centrifugation are pooled together and filtered once again.

The combined aqueous phases are dried under vacuum using a rotary evaporator, and then weighed. Total weight of dry extract: 6.25 g. In order to quantify the tyrosol, hydroxyl tyrosol and lactic acid content, we use syringaldehyde as internal standard and qNMR. The NMR spectrum indicates that this extract contains 7 mg/g tyrosol, 9 mg/g hydroxytyrosol and 51 mg/g lactic acid. No maslinic acid is detected.

To enrich the dried aqueous extract, remove inorganic material (e.g. sodium chloride) and prepare the material for encapsulation, the following procedure is applied: The dry aqueous extract is dissolved in ethanol or isopropanol (1:5 w/v) and the insoluble part is discarded by filtration. The ethanol solution is mixed with microcrystalline cellulose (2:3 w/w dry weight) and dried under vacuum using a rotary evaporator affording a powder that is encapsulated in hard capsules. Each hard capsule has a content of 500 mg including 6 mg tyrosol, 10 mg hydroxytyrosol and 51 mg lactic acid. The preferred daily dose is equivalent to 5 olives is contained in two capsules.

After the three water extractions as described above, the olive fruit paste weighs 146.3 g. The material is dried using an oven at 60 °C for 72 hours, with gentle stirring every 24 hours. After 3 days the dry weight of the product is 46.5 g. The dry product, similar to a cookie, is pulped using a blender to give a semi-liquid texture resembling a jam or cream. In order to quantify the phenolic content of this material, we use a methanol:water extraction, followed by defatting using cyclohexane and internal standard addition. Using qNMR and data processing, the results show that this product contains 0.24 mg/g tyrosol and 0.54 mg/g hydroxytyrosol, as well as 3.81 mg/g lactic acid and 3.23 mg/g maslinic acid.

## Claims

1. A method of producing at least one composition, said method comprising the following steps:
(a) preparing a paste from flesh of fermented Kalamon olives;
(b) subjecting said paste to a separation process yielding oil, a semisolid fraction, and an aqueous phase, said separation process preferably being centrifugation; and
(c) performing one, two or three of the following:
(i) harvesting said oil, thereby obtaining a first composition;
(ii) drying said semisolid fraction, thereby obtaining a second composition; and
(iii) harvesting said aqueous phase, thereby obtaining a third composition.

2. The method of claim 1, further comprising:
(ba) washing, preferably a plurality of times such as three times, said semisolid fraction obtained in (b) with water, thereafter combining said water with said aqueous phase obtained in (b), thereby obtaining a washed and dried second composition in step (c), and a combined aqueous phase yielding the third composition of step (c); and/or
(ca) filtrating said oil obtained in (c)(i), thereby obtaining a filtrated first composition.

3. The method of claim 1 or 2, further comprising:
(d) encapsulating said first composition into a first capsule, wherein preferably
(i) said first capsule is a soft capsule; and/or
(ii) the amount of said first composition which is encapsulated corresponds to two to ten, preferably five of said olives.

4. The method of any one of the preceding claims, further comprising:
(e) encapsulating said second composition into a second capsule, wherein preferably
(i) said second capsule is a hard capsule and/or a capsule with an enteric coating;
and/or
(ii) the amount of said second composition which is encapsulated corresponds to two to ten, preferably five of said olives.

5. The method of any one of the preceding claims, further comprising:
(f) encapsulating said third composition into a third capsule, wherein preferably
(i) said third capsule is a soft capsule; and/or
(ii) the amount of said third composition which is encapsulated corresponds to two to ten, preferably five of said olives.

6. The method of claim 5, to the extent claim 5 refers to claim 3, said method further comprising:
(g) encapsulating said first capsule and said third capsule into a fourth capsule, preferably a hard capsule and/or a capsule with an enteric coating.

7. The method of any one of the preceding claims, further comprising, prior to step (a), allowing fermentation of said olives to occur, wherein said fermentation occurs
(i) for a time span of about 3 to about 12 months, preferably about 4 to about 6 months; and/or
(ii) in the presence of autochthonous yeasts, wherein preferably no further agent triggering fermentation is added.

8. The method of any one of the preceding claims, wherein said Kalamon olives have been grown in Peloponnese, preferably in Lakonia or Messinia.

9. One or more compositions or capsules selected from:
(i) a combination of said first and said third composition, both obtained by the method of any one of the preceding claims, said first and third capsules obtained by the method of claim 3 or 5, respectively, or the fourth capsule obtained by the method of claim 6;
(ii) the first composition or capsule obtained by the method of any one of the preceding claims;
(iii) the second composition or capsule obtained by the method of any one of the preceding claims; and
(iv) the third composition or capsule obtained by the method of any one of the preceding claims.

10. The composition or capsule of claim 9 (i) or (iv), wherein said composition or capsule comprises
(i) phenolic compounds such as hydroxytyrosol and tyrosol, wherein preferably each of hydroxytyrosol and tyrosol is present in a mass ratio of 1 mg/g to 20 mg/g; and/or
(ii) lactic acid, preferably less or equal to 150 mg/g.

11. The composition or capsule of claim 9 (iii), wherein said composition or capsule, if present, comprises triterpenic compounds such as maslinic acid and oleanolic acid, wherein preferably each of maslinic acid and oleanolic acid is present in a mass ratio of 0.1 mg/g to 5 mg/g.

12. A food supplement comprising or consisting of the composition or capsule of claim 9 or 10, preferably of claim 9(i).

13. A composition comprising or consisting of the composition or capsule of any one of claims 9 to 11 for use in medicine, preferably for use in a method of treating
(a) dyslipidemia;
(b) disorders associated with elevated levels of LDL cholesterol;
(c) disorders associated with elevated levels of total cholesterol; and/or
(d) disorders associated with low levels of HDL cholesterol.

14. The composition for use of claim 13, wherein
(a) said levels are concentrations in serum, blood or plasma;
(b) said elevated levels of LDL cholesterol are serum concentrations;
(c) said elevated levels of total cholesterol are serum concentrations above about 200 mg/dl serum, preferably between about 200 mg/dl and about 300 mg/dl serum, more preferably between about 215 mg/dl and 275 mg/dl serum; and/or
(d) the individual to be treated does not receive any further medication for the treatment of hypercholesterolemia such as statins.

15. The composition for use of claim 13 or 14, wherein said disorders are selected from cardiovascular diseases, atherosclerosis, cardiac infarct, stroke, thrombosis and embolism.

16. Use of the food supplement of claim 12 for lowering
(a) lowering
(i) LDL cholesterol; and/or
(ii) total cholesterol;
and/or
(b) increasing HDL cholesterol
in an individual,
wherein preferably
(1) said use is non-therapeutic; and/or
(2) said individual is a healthy individual, does not suffer from, and/or preferably is not at risk to develop any of the disorders defined in claims 13 to 15.
